(19) Europäisches Patentamt
European Patent Office
Office européen des brevets

(11) **EP 1 111 624 B1**

(12) **EUROPÄISCHE PATENTSCHRIFT**

(45) Veröffentlichungstag und Bekanntmachung des Hinweises auf die Patenterteilung:
**14.12.2011 Patentblatt 2011/50**

(51) Int Cl.:
**G21K 1/04** *(2006.01)*     **H05G 1/30** *(2006.01)*

(21) Anmeldenummer: **00204612.6**

(22) Anmeldetag: **20.12.2000**

(54) **Röntgenuntersuchungsgerät**

X-ray examination apparatus

Appareil d'examen radiologique

(84) Benannte Vertragsstaaten:
**DE FR GB NL**

(30) Priorität: **23.12.1999 DE 19962281**

(43) Veröffentlichungstag der Anmeldung:
**27.06.2001 Patentblatt 2001/26**

(73) Patentinhaber:
• **Philips Intellectual Property & Standards GmbH**
**20099 Hamburg (DE)**
Benannte Vertragsstaaten:
**DE**
• **Koninklijke Philips Electronics N.V.**
**5621 BA Eindhoven (NL)**
Benannte Vertragsstaaten:
**FR GB NL**

(72) Erfinder: **Schmitz, Georg, Dr.**
**Habsburgerallee 11,**
**52064 Aachen (DE)**

(74) Vertreter: **Maiwald Patentanwalts GmbH**
**Elisenhof**
**Elisenstrasse 3**
**80335 München (DE)**

(56) Entgegenhaltungen:
| | |
|---|---|
| EP-A- 0 453 027 | EP-A- 0 496 438 |
| EP-A- 0 981 999 | EP-A1- 0 309 813 |
| WO-A2-99/38172 | US-A- 4 028 554 |
| US-A- 4 766 603 | US-A- 4 817 125 |
| US-A- 5 170 425 | US-A- 5 970 112 |

**Beschreibung**

Die Erfindung bezieht sich auf ein Röntgenuntersuchungsgerät mit einer Röntgenquelle, einem Röntgendetektor, mit einem Absorptionsmittel, das zwischen Röntgenquelle und Röntgendetektor angeordnet ist, einer Steuereinheit zur Einstellung der Absorptionsmittel, einer Bildverarbeitungseinheit und einer Anzeigeeinheit.

Absorptionsmittel werden bei Röntgenuntersuchungsgeräten eingesetzt, um den Röntgenstrahlengang zu begrenzen. Dazu sind diese zwischen Röntgenquelle und Röntgendetektor angeordnet. Einerseits wird durch diese Begrenzung des Röntgenstrahlengangs die zu durchleuchtende Region des Patienten auf ein notwendiges Optimum begrenzt. Andererseits wird durch diese Begrenzung das vom Arzt zu diagnostizierende Röntgenbild auf den Bereich begrenzt, der das zu untersuchende Organ darstellt, ohne dass zusätzliche Bereiche dargestellt werden, die keine nützliche Information liefern, sondern das Röntgenbild in seiner Qualität beeinträchtigen könnten.

Ein Röntgenuntersuchungsgerät mit Absorptionsmittel wird in der US 5287396 beschrieben. Dieses Röntgenuntersuchungsgerät umfaßt eine Bildverarbeitungseinheit, die einen Speicher beinhaltet, in dem Absorptionswerte eines Röntgenbildes in Matrixform gespeichert sind. Mittels Detektionsmitteln wird das Röntgenbild in Unterbereiche segmentiert. Anhand eines Schwellenwertes für den Absorptionswert wird das Bild in Vordergrund und Hintergrund eingeteilt. Mittels einer Berechnungseinheit und der Unterteilung wird nun die Position der Absorptionsmittel berechnet. Die Absorptionsmittel werden dann mittels einer Antriebseinheit an die berechnete Position verschoben. Die Absorptionsmittel werden automatisch so positioniert, dass sich eine maximale Fläche des abgedeckten Hintergrundes bei minimaler Fläche des abgedeckten Vordergrundes ergibt.

Nachteilig bei der beschriebenen automatischen Positionierung der Absorptionsmittel ist die Unterteilung oder Segmentierung des Bildes in zwei binäre Klassen. Die Positionierung der Absorptionsmittel wird basierend auf dieser Unterteilung vorgenommen. Diese Art der Segmentierung stellt eine unnötige Einschränkung bei der Berechnung der Position der Absorptionsmittel dar, da nicht nur die lokale Bildhelligkeit für die Positionierung der Absorptionsmittel ausschlaggebend ist. Absorptionsmittel weisen an ihren Rändern häufig eine schwache Dämpfung auf, wodurch bei Abdeckung diagnostisch relevanter Bereiche das Röntgenbild weniger gedämpft wird. Dies wird in der US 5287396 nicht berücksichtigt.

Bei einer vom Arzt vorgenommenen manuellen Einstellung der Absorptionsmittel werden weitere Informationen zur Einstellung genutzt. Letztlich findet der Arzt einen Kompromiß zwischen allen berücksichtigten Parametern.

Die Positionierung erfolgt in heutigen Systemen entweder unmittelbar manuell mechanisch oder durch motorische Positionierung an eine manuell nach Bildeindruck gewählte Position. Diese manuelle Einstellung benötigt Untersuchungszeit, ist für den Arzt störend und lenkt von der eigentlichen Untersuchung und der Betreuung des Patienten ab.

Daher ist es Aufgabe der Erfindung, ein Röntgenuntersuchungsgerät anzugeben, bei dem die Absorptionsmittel automatisch optimal eingestellt sind.

Diese Aufgabe wird durch ein Röntgenuntersuchungsgerät gemäß Anspruch 1 gelöst.

Die von der Röntgenquelle ausgestrahlte Röntgenstrahlung passiert die Absorptionsmittel und durchleuchtet den Patienten. Auf einem Röntgendetektor wird das durch die Röntgenstrahlung erzeugte Röntgenbild dargestellt. Dieses Röntgenbild wird nach einer Umwandlung in ein elektrischen Bildsignal zu einer Bildverarbeitungseinheit übertragen und dort gespeichert, gleichzeitig wird es auf einem Monitor dargestellt.

Bei medizinischen Röntgenuntersuchungen werden bei einer Röntgenaufnahme die Absorptionsmittel so positioniert, dass die Strahlung in Bildbereichen, in denen der Detektor von direkter, nicht durch den Patienten geschwächter Röntgenstrahlung getroffen wurde, abgeschwächt oder ausgeblendet wird, um störende Überstrahlungen zu vermeiden, die Röntgenstrahlung in diagnostisch irrelevanten Bereichen abgeschwächt wird, um unnötige Patientendosis und Streustrahlung zu vermeiden, die Röntgenstrahlung in diagnostisch relevanten Bereichen, die z.B. anatomische Strukturen enthalten, nicht ausgeblendet wird, um einen optimalen diagnostischen Nutzen zu ermöglichen. Diese Maßnahmen führen zu einer Optimierung der Bildqualität und der Patientendosis.

Für die Einstellung der Absorptionsmittel lassen sich folgende Parameter klassifizieren. Basis für eine optimale Einstellung ist die Vorstellung des diagnostizierenden Arztes wie das dargestellte Röntgenbild auszusehen hat. Diese Vorstellung wird in den benutzerspezifischen Parametern zusammengefaßt. Die gerätespezifischen Parameter beinhalten Angaben, wie Art des Röntgenuntersuchungsgerätes, Röhrenspannung, Röhrenstrom und Belichtungszeit. Strukturparameter sind Werte, die aus dem Röntgenbild ermittelt werden. Strukturparameter enthalten Angaben zu den Graustufenwerteverlauf über eine Gruppe von Bildpixeln. Strukturparameter enthalten ebenso Angaben über den Bildkontrast. Dazu wird über die Graustufenwerte des gesamten Bildes ein Histogramm erstellt, in dem das Auftreten der jeweiligen Graustufenwerte aufgezeigt wird. Die sich dabei ergebende Verteilung kann als Maß für den Kontrast verwendet werden.

Die den Gegenstand der Aufnahme klassifizierenden Parameter enthalten Angaben über das Organ oder die Körperregion, die mittels Röntgenstrahlung durchleuchtet werden soll. Die benutzerspezifischen Parameter und die den Gegenstand der Aufnahme klassifizierenden Parameter werden als Wissensbasis zusammengefaßt.

In der Bildverarbeitungseinheit sind Rechenmittel angeordnet, denen diese Parameter zugeführt werden und die anhand dieser Parameter eine optimale Einstellung der Absorptionsmittel berechnen. Diese Parameter sind in Speichern gespeichert oder werden aus dem aufgenommen Röntgenbild extrahiert. Die Parameter werden in einer Gütefunktion zusammengefaßt, die in vorzugebender Weise optimiert wird. Die auf diese Weise berechnete Einstellung wird der Steuereinheit zugeführt, über die die Absorptionsmittel in die berechnete Position gebracht werden oder mittels der die Absorptionsmittel die berechnete Einstellung einnehmen.

In einer Ausführung des erfindungsgemäßen Röntgenuntersuchungsgerätes sind die Absorptionsmittel für Röntgenstrahlung im wesentlichen nicht transparent. Die Absorptionsmittel können vorteilhafterweise eine nicht hundertprozentige Absorption hervorrufen, so dass auf dem Röntgenbild ein sanfter Übergang zwischen belichtetem Bereich und abgedecktem Bereich entsteht. Dazu ist beispielsweise die Form der Absorptionsmittel keilförmig, so dass sich an der Spitze des Keils eine nicht vollständige Absorption ergibt. Auch dieser Verlauf der Dämpfung geht mit in die Wissensbasis ein.

Die Absorptionsmittel können in einer Blendenvorrichtung so angeordnet sein, dass sie mittels Schiebevorrichtung, die elektrisch oder hydraulisch angetrieben werden, in die berechnete Position gebracht werden. Hierbei werden die oben beschriebenen Keile oder Plattenblenden verwendet. Die Absorptionsmittel sind so angeordnet, dass sie den kegelförmigen Röntgenstrahlengang von allen Seiten begrenzen können oder durch partielles Einschieben einer Einzelblende nur an einer Stelle den Röntgenstrahlengang begrenzen.

In einer weiteren Ausführungsform des erfindungsgemäßen Röntgenuntersuchungsgerätes wird ein aus einer Vielzahl füllbarer Filterelemente bestehendes Filter als Absorptionsmittel eingesetzt. Hierbei kann der Füllungsgrad einer Röntgenstrahlen dämpfenden Flüssigkeit elektrisch eingestellt werden. So kann eine über den Strahlenquerschnitt variierende Röntgendämpfung realisiert werden.

Als vorteilhaft erweist es sich, eine berechnete Einstellung der Absorptionsmittel der Darstellung des Röntgenbildes auf dem Monitor zu überlagern, bevor die Einstellung definitiv mittels der Steuereinheit vorgenommen wird. Der untersuchende Arzt kann die berechnete Einstellung somit werten und gegebenenfalls korrigieren.

Ebenso erweist es sich als vorteilhaft, den Verlauf der Parameteränderung zu speichern, um Erfahrungswerte zu sammeln und anhand dieser Erfahrungswerte die Parameter der Wissensbasis zu adaptieren. Der Grad der Adaption kann dabei eingestellt werden. Dadurch wird ein Lernprozeß realisiert, der letztlich eine schnellere, automatische und korrektere Einstellung der Absorptionsmittel ermöglicht.

Im folgenden wird beschrieben, wie die Parameter in einer Gütefunktion $Z$ zusammengefaßt werden und einer Optimierung unterzogen werden. Die Parameter für die Einstellung der Absorptionsmittel werden in einem Einstellungsparametervektor $\underline{p}_{akt}$ gespeichert. Dies können zum Beispiel ein Winkel und die Einschublänge einer semitransparenten Blende sein.

Durch eine Simulation, z.B. mit Röntgenstrahlverfolgung, kann die Dämpfungswirkung der im aktuellen Röntgenbild angewendeten Einstellung der Absorptionsmittel berechnet und durch Division aus dem in der Bildverarbeitungseinheit gespeichertem Röntgenbild entfernt werden.

Aus dem Ergebnisbild werden in der Bildverarbeitungseinheit für $N$ verschiedene Bildbereiche $M$ den Bildinhalt charakterisierende Parameter berechnet. In der Regel werden diese Bildbereiche lückenlos oder überlappend aneinandergefügte Rechtecke sein. Die das Bild charakterisierenden Parameter sind z.B. die mittlere Helligkeit oder Maße für Bildkontrast und enthaltene Struktur. Alle berechneten Parameter werden in einer $M \times N$ Parametermatrix $\underline{C}$ zusammengefaßt. Der Kontrast kann beispielsweise mittels Varianzberechnung ermittelt werden. Auch eine Parameterberechnung basierend auf Haralick (IEEE 67(5): 610-621,1979), in der statistische und strukturelle Verfahren zur Textur oder Beschaffenheit von Bildern beschrieben werden, können verwendet werden.

Alternativ zu dieser direkten Berechnung der Parameter können die Parameter der Matrix $\underline{C}$ aus dem im Bildspeicher abgelegten Bild direkt berechnet werden und die Auswirkung der aktuellen Dämpfung durch die Absorptionsmittel durch eine Korrektur der Parameter berücksichtigt werden. Bei dem obengenannten Beispielparameter der mittleren Helligkeit ist die Division der mittleren Helligkeit durch die mittlere Dämpfung eine solche Korrekturmöglichkeit.

Nun wird die Dämpfung $D$ der Absorptionsmittel in Abhängigkeit möglicher neuer Einstellungsparameter berechnet. Die zu testenden neuen Einstellungsparameter sind im Einstellungsparametervektor $\underline{p}$ zusammengefaßt.

Die Parametermatrix $\underline{C}$ und die Dämpfung $D$ können nun zur Beurteilung der Güte der Wirkung der Absorptionsmittel herangezogen werden. Hierzu wird eine geeignete Gütefunktion $Z$ berechnet, die durch mehrere die gewünschte Bildqualität charakterisierende Faktoren parametrisiert sein kann, wie z.B. durch Gewichtungen für verschiedene Bildqualitätsmaße oder Parameter. Die Parameter der Gütefunktion Z, die das Verhalten der Funktion $Z$ in Abhängigkeit von den Funktionsargumenten charakterisieren, werden im Vektor $\underline{r}$ zusammengefaßt. In den Vektor $\underline{r}$ gehen die benutzerspezifischen Parameter und die den Gegenstand der Aufnahme klassifizierenden Parameter ein. Benutzerspezifische Parameter enthalten beispielsweise Vorlieben bestimmter Bediener bei der Darstellung des teilweise abgedeckten Röntgenbildes. Die den Gegenstand der Aufnahme charakterisierenden Parameter enthalten Angaben über die Aufnahmeart. Bei einem Röntgenbild des Herzen wird beispielsweise um das zu diagnostizierende Herz die sehr hell dargestellte Lunge abgebildet. Dieses Wissen geht in die den Gegenstand der Aufnahme klassifizierende Parameter ein, die wie die benutzerspezifischen Parameter im Vektor $\underline{r}$, der die Wissensbasis darstellt, enthalten sind.

Die gerätespezifischen Parameter werden im Systemparametervektor $\underline{s}$ zusammengefaßt. Der aktuelle Zustand des Röntgenuntersuchungsgerätes oder hieraus errechnete Parameter fließen in den Systemparametervektor $\underline{s}$ ein. Hierbei kann es sich z.B. um die im aktuellen Bild applizierte Röntgendosis handeln. Ebenso kann als abgeleiteter Parameter eine dosisabhängige Grauwertschwelle für überstrahlte Bereiche in diesen Systemparametervektor $\underline{s}$ eingehen.

Die Gütefunktion Z kann entweder heuristisch, oder über regelbasierte Methoden, wie z.B. die der unscharfen Logik abgeleitet werden. Ebenso ist es möglich, die Gütefunktion als neuronales Netz zu realisieren, das vor Inbetriebnahme des Röntgenuntersuchungsgerätes an repräsentativen Bilddaten und gewünschten Einstellungen der Absorptionsmittel trainiert wird.

Allgemeiner kann jeder lineare oder nichtlineare Funktionenapproximator, der auf der Basis von Trainingseingangs- und Trainingsausgangsdaten das erwünschte Verhalten annähert, verwendet werden. Der Vektor $\underline{r}$ parametrisiert die Einstellregel und stellt somit die Wissens- oder Regelbasis des Systems dar.

Zur Berechnung einer optimalen Blendenposition oder optimalen Einstellung der Absorptionsmittel wird ein Optimierungsverfahren verwendet, dass den Einstellungsparametervektor $\underline{p}$ variiert und jeweils die Dämpfung D und daraus den Funktionswert Z(D,r,s) ermittelt. Als Optimierungsverfahren kann z.B. ein einfacher Algorithmus, wie das Nelder-Mead Simplex Verfahren (Nelder, J.A. and Mead,R. 1965, Computer Journal, vol. 7,pp.308-313) benutzt werden. Ergebnis der Optimierung ist die optimierte Einstellung der Absorptionsmittel, die im Einstellungsparametervektor $\underline{p}_{opt}$ erfaßt ist.

$$\underline{p}_{opt} = \arg\operatorname*{opt}_{\underline{p}} Z(r,s,\underline{\underline{C}},D(\underline{p})) \tag{1}$$

Diese Einstellung der Absorptionsmittel kann direkt automatisch über eine Steuereinheit realisiert werden. Alternativ können die Absorptionsmittel in Form von Blenden oder Blendenkanten dem Monitorbild grafisch überlagert werden. Der Bediener hat so die Möglichkeit zur Korrektur und Bestätigung. Die neue aktuelle Einstellung $\underline{p}_{akt}$ kann wiederum verwendet werden, die Wissensbasis, die in Vektor $\underline{r}$ repräsentiert wird, an das Benutzerverhalten zu adaptieren. Dies kann entweder über ein erneutes Trainieren des verwendeten lernenden Systems geschehen. Ebenso ist folgende einfache Adaption der Regelbasis möglich:

Für die korrigierte Einstellung $\underline{p}_{akt}$ wird eine Optimierung der Funktion Z in Abhängigkeit von der Wissensbasis $\underline{r}$ durchgeführt:

$$\underline{r}_{opt} = \arg\operatorname*{opt}_{\underline{r}} Z(\underline{r},\underline{s},\underline{\underline{C}},D(\underline{p}_{akt})) \tag{2}$$

Je nach Gütefunktion kann die Optimierung eine Maximierung oder Minimierung sein. Die neue Regelbasis $\underline{r}'$ wird dann über einen Lernschritt ermittelt:

$$\underline{r}' = \underline{r} + \alpha(\underline{r}_{opt} - \underline{r}) \tag{3}.$$

Hierin gibt $\alpha$ die Lernrate an, die im Intervall [0,1] liegt. Für $\alpha = 1$ wird das System sprungartig angepaßt, für $\alpha = 0$ ist das System nicht adaptiv. In der Regel werden kleine Werte für $\alpha$ verwendet. Die Anpassung erfolgt benutzerspezifisch und wird für verschiedene Benutzer und Applikationen in einer Datenbank gespeichert. Derartige Parameter fließen in den Vektor r ein.

Gegenüber dem im Stand der Technik beschriebenen Röntgenuntersuchungsapparat kann das erfindungsgemäße Röntgenuntersuchungsgerät durch den Wegfall einer binären Segmentierung in Hintergrund und Bildinhalt und die statt dessen durchgeführte Bewertung der Gesamtbildqualität eines virtuellen Bildes mit positionierter Blende auch die Qualität von Teilüberdeckungen relevanter Objekte beurteilen. So kann der Bediener z.B. bei geeigneter Wahl der Gütefunktion den erlaubten Überdeckungsgrad über die Einstellung nur eines Parameters festlegen. Diese Einstellung kann benutzer- und applikationsspezifisch adaptiert und gespeichert werden.

Ein Ausführungsbeispiel der Erfindung wird im folgenden anhand der Zeichnungen näher erläutert. Es zeigen:

Fig. 1    schematische Darstellung eines Röntgenuntersuchungsgerätes

Fig. 2    Aufbau der Bildverarbeitungseinheit

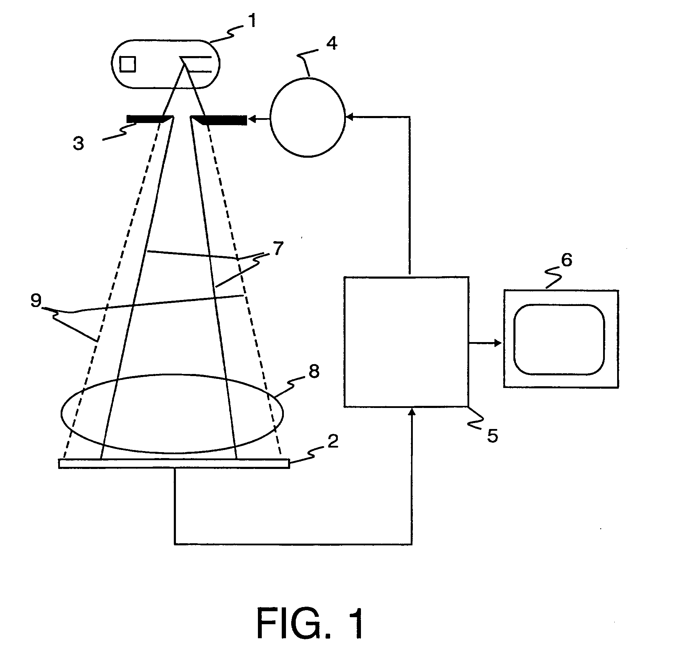

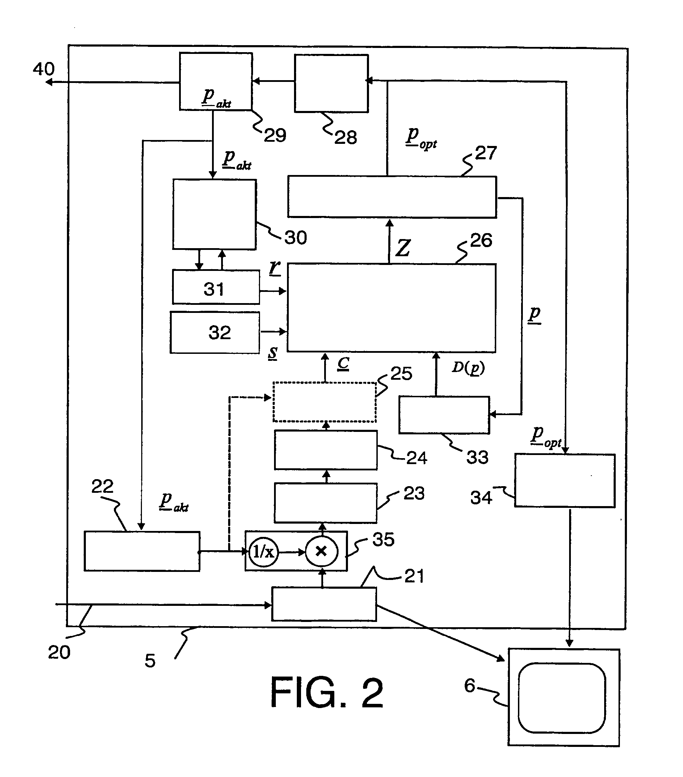

Die Figur 1 zeigt den Aufbau des erfindungsgemäßen Röntgenuntersuchungsgerätes. Die Röntgenstrahlungsquelle 1 emittiert Röntgenstrahlung 9. Diese Röntgenstrahlung passiert die Absorptionsmittel 3, die im folgenden auch als Kollimatorblenden 3 bezeichnet werden. Die Kollimatorblenden 3 werden von der Steuereinheit 4 verschoben. Die von den Kollimatorblenden 3 durchgelassene Röntgenstrahlung 7 durchleuchtet das zu untersuchende Objekt 8 und trifft auf den Röntgendetektor 2. Dort wird das Röntgenbild aufgenommen und in ein elektrisches Bildsignal umgewandelt. Dieses Bildsignal 20 wird der Bildverarbeitungseinheit 5 zugeführt. Das aufgenommene Bild wird auf der als Monitor 6 realisierten Anzeigeeinheit dargestellt. Die Bildverarbeitungseinheit 5 ist mit der Steuereinheit 4 zur Einstellung des Absorptionsgrades der Kollimatorblenden 3 verbunden. In Figur 2 wird der Aufbau der Bildverarbeitungseinheit 5 dargestellt. Der Bildverarbeitungseinheit 5 wird das Bildsignal 20 vom hier nicht dargestellten Röntgendetektor 2 zugeführt. Dort wird es dem Bildspeicher 21 zur Speicherung zugeführt und gleichzeitig dem Monitor 6 zur Darstellung zugeführt. In der Dämpfungsberechnungseinheit 22 wird mittels des im Speicher 29 abgelegten Einstellungsparametervektor $\underline{p}_{akt}$ die aktuelle Dämpfung berechnet. Die aktuelle Dämpfung $D(\underline{p}_{akt})$ ist der Wert der Dämpfung der dem aufgenommenen Röntgenbild anhaftet, da Absorptionsmittel eventuell teilweise in den Röntgenstrahlengang eingeschoben sind. In der Einheit 35 wird von dem aktuellen Dämpfungswert $D$ der reziproke Wert gebildet, um diesen dann mit dem im Bildspeicher 21 abgelegten Röntgenbild zu multiplizieren und somit ein von der Dämpfung $D$ bereinigtes Bild zu erhalten.

In der Einheit 23 wird das ungedämpfte Bild in Bildbereiche N unterteilt wird. Dieser Einheit 23 nachfolgend ist eine Parameterextraktionseinheit 24 zur Berechnung der Bildparameter, wie Kontrast- oder Strukturparameter angeordnet. In der optionalen Dämpfungskorrektureinheit könnte anhand des Einstellparametervektors $\underline{p}_{akt}$ auch die Dämpfung herausgerechnet werden. Die so gebildete Parametermatrix $\underline{C}$ wird der Gütefunktionsberechnungseinheit 26 zugeführt. Dieser Einheit 26 werden die Systemparameter aus der Einheit 32 in Form des Systemparametervektors $\underline{s}$ und des die Wissensbasis darstellenden Vektors $\underline{r}$ zugeführt. Die Wissens- oder Regelbasis in Form des Vektor $\underline{r}$ ist in Einheit 31 abgelegt und wird sowohl der Berechnung der Gütefunktion $Z$ in Einheit 26 zugeführt, als auch der Adaption der Wissensbasis in der Adaptionseinheit 30.

Die Gütefunktion wird in der Einheit 27 optimiert. Es wird der Einstellungsparametervektor $\underline{p}_{opt}$ berechnet, der die optimierte Einstellung der Absorptionsmittel beinhaltet.

Dieser Einstellungsparametervektor $\underline{p}_{opt}$ wird einer weiteren Dämpfungskorrektureinheit 33, die der Dämpfungseinheit 22 gleicht, zugeführt, die anhand des Einstellungsparametervektor $\underline{p}_{opt}$ die Einstellung der Dämpfung $D(\underline{p})$ berechnet, um diese wiederum der Berechnung der Gütefunktion in Einheit 26 zuzuführen und in einem nächsten Durchlauf den Einstellungsparametervektor $\underline{p}$ weiter zu optimieren, bis der optimierte Einstellungsparametervektor $\underline{p}_{opt}$ ermittelt wird. Dieser wird dann der Einheit 28 zugeführt, in der überprüft bzw. bestätigt wird, ob die momentane Einstellung der Blenden, die auch dem Monitor 6 zugeführt wird, korrekt ist. Wird hier die momentane Einstellung in Form des Einstellungsparametervektor $\underline{p}_{opt}$ vom Arzt bestätigt, wird der Einstellungsparametervektor $\underline{p}_{akt}$ im Speicher 29 gespeichert und von dort zur Steuereinheit, die hier nicht dargestellt ist, weitergeleitet. Gleichzeitig wird die im Einstellungsparametervektor $\underline{p}_{opt}$ verkörperte Einstellung der Absorptionsmittel in der Überlagerungseinheit 34 dem aktuellen Röntgenbild überlagert.

Vom Speicher 29, in dem der aktuell angewendete Einstellungsparametervektor $\underline{p}_{akt}$ abgelegt ist, wird dieser $\underline{p}_{akt}$ an die Adaptionseinheit 30 weitergeleitet, in der mittels Lernprozeß die Wissensbasis adaptiert wird.

Ein einfaches Beispiel soll die Anwendung der Gütefunktionsberechnung verdeutlichen.

Das im Bildspeicher abgelegte Bild $I(x, y)$ wird in $N$ nicht überlappende, rechteckige Bereiche $I_i (x, y)$ von $N_x$ Pixeln Breite und $N_y$ Pixeln Länge aufgeteilt. Für diese Bereiche wird die enthaltene Struktur durch die Varianz beschrieben und mit

$$\sigma_i^2 = \frac{1}{(N_x N_y - 1)} \sum_{x=1}^{N_x} \sum_{y=1}^{N_y} \left( I_i (x,y) - \frac{1}{N_x N_y} \sum_{x=1}^{N_x} \sum_{y=1}^{N_y} I_i (x,y) \right)^2 \qquad (4)$$

als Parameter berechnet. Da nur ein Parameter für alle Bildbereiche berechnet wird, hat die Parametermatrix $\underline{C}$ nur eine Zeile:

$\underline{C} = (\sigma_1 \cdots \sigma_N$

Für ein einzelnes Absorptionsmittel, im folgenden eine semitransparente Blende, deren Position durch den Einstellungsparametervektor $\underline{p} = (l, \varphi)$ mit Winkel $\varphi$ und Einschublänge $l$ gekennzeichnet ist, kann die Auswirkung der Strahlabschwächung auf ein Bild $I$ durch eine Funktion $D(p,x,y)$ so angegeben werden, dass das abgeschwächte Bild $I_D$ durch

$$I_D(\underline{p},x,y) = D(\underline{p},x,y)I(x,y) \qquad (5)$$

gegeben ist. $D(\underline{p}, x, y)$ kann z.B. über einfache Simulationen näherungsweise bestimmt werden.

Nun ist für die Optimierung eine Gütefunktion $Z(\underline{r}, \underline{s}, \underline{\underline{C}}, D(\underline{p}))$ anzugeben, die von den zusätzlichen Parametervektoren $\underline{r}$ und $\underline{s}$ abhängt. Ein einfaches Beispiel einer solchen Gütefunktion kann durch die gewichtete Addition mehrerer Einzelgütefunktionen gegeben werden: Sollen zum Beispiel im wesentlichen Bereiche mit geringer Struktur beseitigt werden, so kann durch einen Anteil die Abdämpfung strukturhaltiger, diagnostisch relevanter Bereiche bestraft werden, d.h. solche Bereiche führen zu einer Erhöhung der Gütefunktion, die dann zu minimieren ist. Ein weiterer Anteil bestraft strukturarme Bereiche, die nicht unter die eingeschobene Blende gefallen sind. Eine solche Gütefunktion stellt z.B. die folgende dar:

$$Z(r,s,\underline{\underline{C}},D(\underline{p})) = \sum_{n=1}^{N} \left( rU(D_n(\underline{p})(1 - c_{1,n}/s^2)) + U((1 - D_n(\underline{p}))(c_{1,n}/s^2 - 1)) \right) \qquad (6)$$

mit

$$D_n = \frac{1}{N_x N_y} \sum_{x=1}^{N_x} \sum_{y=1}^{N_y} D(\underline{p},x,y) \qquad (7)$$

und den Elementen von $\underline{\underline{C}}$ gemäß

$$c_{1,n} = \{\underline{\underline{C}}\}_{1,n} .$$

In diesem Beispiel hat die Regelbasis nur eine Komponente $r$; der Parameter $s$ stellt die einzige Komponente des Systemparametervektors $\underline{s}$ dar und gibt eine dosis- und strahlqualitätsabhängige Normierung des diagnostisch relevanten Kontrastes. Dieser Parameter kann in Abhängigkeit der verwendeten Strahlqualität aus einer gespeicherten Tabelle ausgelesen werden. Die Funktion U bestraft zunehmend positive Beiträge. Sie kann z.B. durch

$$U(x) = \begin{cases} x^2 & \text{für } x > 0 \\ 0 & \text{für } x \leq 0 \end{cases}$$

gegeben sein.

Der erste Term der Gütefunktion (6) bestraft somit unstrukturierte Bereiche, die nicht bedämpft werden. Der zweite Term der Gütefunktion (6) bewertet den Effekt der eingebrachten Blende auf Kontraste im diagnostisch relevanten Bereich. Beide Anteile sind widersprüchlich und werden über $r$ gegeneinander gewichtet. Je größer $r$ gewählt wird, um so mehr wird der Unterdrückung unstrukturierter Bereiche gegenüber dem Erhalt diagnostisch relevanter Information Vorrang gegeben.

Die optimale Blendenposition wird nun über die Minimierung der Gütefunktion bestimmt:

$$\underline{p}_{opt} = \arg\min_{\underline{p}} Z(r,s,\underline{\underline{C}},D(\underline{p})). \qquad (8)$$

Dies kann z.B. numerisch über den Nelder-Mead Simplex Algorithmus erfolgen. Die so bestimmte Blendenpostion wird dann grafisch auf dem Monitor überlagert oder direkt motorisch eingestellt. Die Korrektur kann, wie bereits beschrieben, benutzt werden, die benutzer- oder applikationsspezifische Anpassung von *r* vorzunehmen.

**Patentansprüche**

1. Röntgenuntersuchungsgerät mit einer Röntgenquelle (1), einem Röntgendetektor (2), mit einem Absorptionsmittel (3), das zwischen Röntgenquelle und Röntgendetektor angeordnet ist, einer Steuereinheit (4) zur Einstellung der Absorptionsmittel, einer Bildverarbeitungseinheit (5), die Rechenmittel (27) enthält, und einer Anzeigeeinheit (6), **dadurch gekennzeichnet,**
   **dass** mittels der Rechenmittel vorgesehen ist, Parameter einschließlich

   - benutzerspezifischen Parametern ($\underline{r}$) und/oder
   - gerätespezifischen Parametern ($\underline{s}$) und/oder
   - Strukturparametern ($\underline{C}$) und/oder
   - den Gegenstand der Aufnahme klassifizierenden Parametern ($\underline{r}$),

   in einer Gütefunktion zusammenzufassen und für eine optimierte Einstellung der Absorptionsmittel diese Gütefunktion in vorgegebener Weise zu optimieren.

2. Röntgenuntersuchungsgerät nach Anspruch 1,
   **dadurch gekennzeichnet,**
   **dass** die Absorptionsmittel (3) im wesentlichen nicht transparent für Röntgenstrahlung sind.

3. Röntgenuntersuchungsgerät nach Anspruch 1,
   **dadurch gekennzeichnet,**
   **dass** als Absorptionsmittel (3) eine Röntgenstrahlung absorbierende Blendenvorrichtung (3) angeordnet ist.

4. Röntgenuntersuchungsgerät nach Anspruch 1,
   **dadurch gekennzeichnet,**
   **dass** als Absorptionsmittel (3) ein aus einer Vielzahl von mit Röntgenstrahlung absorbierender Flüssigkeit füllbaren Filterelemente angeordnet ist.

5. Röntgenuntersuchungsgerät nach Anspruch 1,
   **dadurch gekennzeichnet,**
   **dass** ein Speicher (29) zur Speicherung von optimierten Parametern ($\underline{p}_{akt}$) vorgesehen ist.

6. Röntgenuntersuchungsgerät nach Anspruch 1,
   **dadurch gekennzeichnet,**
   **dass** eine Überlagerungseinheit (34) vorgesehen ist, die eine berechnete Einstellung der Absorptionsmittel einem darzustellenden Röntgenbild überlagert, bevor die Einstellung der Absorptionsmittel vorgenommen ist.

7. Röntgenuntersuchungsgerät nach Anspruch 1,
   **dadurch gekennzeichnet,**
   **dass** eine Adaptionseinheit (30) zur Adaption der Parameter ($\underline{r}$) über mehrere Röntgenaufnahmen vorgesehen ist.

8. Röntgenuntersuchungsgerät nach Anspruch 1,
   **dadurch gekennzeichnet,**
   **dass** eine Korrektureinheit (28) zur manuellen Korrektur der Einstellung der Absorptionsmittel vorgesehen ist.

9. Röntgenuntersuchungsgerät nach Anspruch 1,
   **dadurch gekennzeichnet,**
   **dass** eine Parameterextraktionseinheit (24) zur Zuführung des dämpfungsbereinigten Bildes und zur Extraktion der das Bild charakterisierenden Parameter($\underline{C}$), wie Kontrast und/ oder Strukturgehalt vorgesehen ist.

## Claims

1. X-ray examination apparatus having an X-ray source (1), an X-ray detector (2), moreover with an absorption means (3) that is arranged between X-ray source and X-ray detector, a control unit (4) for adjusting the absorption means, an image processing unit (5) that contains calculating means (27), and a display unit (6), **characterised in that** it is provided by means of the calculating means to summarise parameters including

   • user-specific parameters ($\underline{r}$) and/or
   • device-specific parameters ($\underline{s}$) and/or
   • structural parameters ($\underline{C}$) and/or
   • parameters ($\underline{r}$) classifying the subject matter of the image, into a quality function and to furthermore optimise said quality function in a pre-determined manner for an optimised adjustment of the absorption means.

2. X-ray examination apparatus as set forth in claim 1,
   **characterised in that**
   the absorption means (3) are substantially not transparent for X-rays.

3. X-ray examination apparatus as set forth in claim 1,
   **characterised in that**
   an X-ray-absorbing diaphragm (3) is arranged as absorption means (3).

4. X-ray examination apparatus as set forth in claim 1,
   **characterised in that**
   a plurality of filter elements that can be filled with X-ray absorbing fluid is arranged as absorption means (3).

5. X-ray examination apparatus as set forth in claim 1,
   **characterised in that**
   memory (29) is provided for storing optimised parameters ($\underline{p_{akt}}$).

6. X-ray examination apparatus as set forth in claim 1,
   **characterised in that**
   a superimposing device (34) is provided that superimposes a calculated adjustment of the absorption means onto an X-ray image to be displayed prior to the adjustment of the absorption means having been undertaken.

7. X-ray examination apparatus as set forth in claim 1,
   **characterised in that**
   an adapter unit (30) is provided for adapting the parameters (r) of a plurality of X-ray images.

8. X-ray examination apparatus as set forth in claim 1,
   **characterised in that**
   a correction unit (28) is provided for manual correction of the adjustment of the absorption means.

9. X-ray examination apparatus as set forth in claim 1,
   **characterised in that**
   a parameter extraction unit (24) is provided for supplying the steam-cleaned image as well as for extracting the parameters (C) characterising the image such as contrast and/or structural content.

## Revendications

1. Appareil d'examen radiologique avec une source de rayons X (1), un détecteur de rayons X (2), avec un moyen d'absorption (3), qui est disposé entre la source de rayons X et le détecteur de rayons X, une unité de commande (4) pour le réglage des moyens d'absorption, une unité de traitement d'image (5), qui comprend des moyens de calcul (27) et une unité d'affichage (6), **caractérisé en ce que**, grâce aux moyens de calcul, il est prévu de réunir dans une fonction caractéristique de fonctionnement des paramètres incluant :

   • des paramètres spécifiques à l'utilisateur (r) et / ou

• des paramètres spécifiques à l'appareil (s) et / ou

• des paramètres structurels (C) et / ou

• l'objet de la réception des paramètres de classification ($\underline{r}$), et d'optimiser cette fonction caractéristique de fonctionnement d'une manière préétablie pour un réglage optimisé des moyens d'absorption.

2. Appareil d'examen radiologique selon la revendication 1,
   **caractérisé en ce**
   **que** les moyens d'absorption (3) sont pour l'essentiel non-transparents aux rayons X.

3. Appareil d'examen radiologique selon la revendication 1,
   **caractérisé en ce**
   **qu'**un mécanisme de diaphragme (3) absorbant les rayons X est disposé comme moyen d'absorption (3).

4. Appareil d'examen radiologique selon la revendication 1,
   **caractérisé en ce**
   **qu'**une multiplicité d'éléments de filtre pouvant être remplis de liquide absorbant les rayons X est disposée comme moyen d'absorption (3).

5. Appareil d'examen radiologique selon la revendication 1,
   **caractérisé en ce**
   **qu'**une mémoire (29) est prévue pour le stockage des paramètres optimisés ($\underline{p_{act}}$)

6. Appareil d'examen radiologique selon la revendication 1,
   **caractérisé en ce**
   **qu'**une unité de superposition (34) est prévue, qui superpose un réglage calculé des moyens d'absorption sur l'image aux rayons X qui doit être présentée, avant d'entreprendre le réglage des moyens d'absorption.

7. Appareil d'examen radiologique selon la revendication 1,
   **caractérisé en ce**
   **qu'**une unité d'adaptation (30) est prévue pour l'adaptation des paramètres (r) sur plusieurs prises d'image aux rayons X.

8. Appareil d'examen radiologique selon la revendication 1,
   **caractérisé en ce**
   **qu'**une unité de correction (28) est prévue pour la correction manuelle du réglage des moyens d'absorption.

9. Appareil d'examen radiologique selon la revendication 1,
   **caractérisé en ce**
   **qu'**une unité d'extraction de paramètres (24) est prévue pour l'alimentation de l'image purifiée par atténuation et pour l'extraction des paramètres ($\underline{C}$) qui caractérisent l'image, comme le contraste et / ou le contenu structurel.

# FIG. 1

# FIG. 2

**EP 1 111 624 B1**

**IN DER BESCHREIBUNG AUFGEFÜHRTE DOKUMENTE**

**In der Beschreibung aufgeführte Patentdokumente**

- US 5287396 A

**In der Beschreibung aufgeführte Nicht-Patentliteratur**

- *IEEE,* 1979, vol. 67 (5), 610-621

- **Nelder, J.A. ; Mead,R.** *Computer Journal,* 1965, vol. 7, 308-313